# EUROPÄISCHE PATENTSCHRIFT

(11) **EP 2 498 762 B1**
(45) Veröffentlichungstag und Bekanntmachung des Hinweises auf die Patenterteilung: **07.09.2016**
(21) Anmeldenummer: 10773582.1
(22) Anmeldetag: 26.10.2010
(51) Int. Cl.: A61K 9/70, A61K 31/381

(54) **VERFAHREN ZUR VERHINDERUNG DER KRISTALLISATION VON ARZNEISTOFFEN IN EINEM POLYMERFILM**
METHOD FOR PREVENTING THE CRYSTALLISATION OF PHARMACEUTICALS IN A POLYMER FILM
PROCÉDÉ POUR EMPÊCHER LA CRISTALLISATION DE SUBSTANCES PHARMACEUTIQUES DANS UN FILM POLYMÈRE

(30) Priorität: 12.11.2009 DE 102009052972
(43) Veröffentlichungstag der Anmeldung: 19.09.2012
(73) Patentinhaber: LTS LOHMANN Therapie-Systeme AG, 56626 Andernach (DE)
(72) Erfinder: LEONHARD, Johannes, Josef, 56170 Bendorf (DE); MÜLLER, Walter, 56626 Andernach (DE)
(74) Vertreter: Schweitzer, Klaus
(86) Internationale Anmeldenummer: PCT/EP2010/006535
(87) Internationale Veröffentlichungsnummer: WO 2011/057714

(56) Entgegenhaltungen:
- EP-A1- 1 669 063
- WO-A1-2004/058247
- US-A- 5 662 928
- US-A1- 2008 226 698

## Beschreibung

Die vorliegende Erfindung betrifft ein Verfahren zur Verhinderung der spontanen Kristallisation eines Arzneistoffs in einem Polymerfilm. Unter einer "spontanen Kristallisation" wird dabei eine Kristallisation verstanden, die ohne einen erkennbaren Stimulus erfolgt.

Transdermale therapeutische Systeme sind Darreichungsformen für eine perkutane Verabreichung von Arzneistoffen. Bei transdermalen therapeutischen Systemen wird zwischen so genannten Beutelsystemen und Matrixsystemen unterschieden. Bei den Beutelsystemen ist der Arzneistoff in Form einer flüssigen oder halbflüssigen Zubereitung in einem flachen Beutel enthalten, dessen Wand eine Membran umfaßt, über die der im Beutel befindliche Arzneistoff abgegeben werden kann. Matrixsysteme zeichnen sich dadurch aus, daß der Arzneistoff in einem Polymerfilm enthalten ist. Matrixsysteme bestehen in der einfachsten Ausführungsform aus einer arzneistoffundurchlässigen Rückschicht, einer arzneistoffhaltigen Matrixschicht, die üblicherweise selbstklebend ist, und einer vor Gebrauch zu entfernenden Schutzschicht. Es gibt aber auch komplexer aufgebaute Matrixsysteme, die mehrere Matrixschichten unterschiedlicher Zusammensetzung, eine zusätzliche Kontrollmembran und/oder auch nicht selbstklebende Schichten aufweisen können.

Arzneistoffhaltige Polymerfilme sind darüber hinaus auch für oral anzuwendende Medikamente in Gebrauch, beispielsweise in Form von film- oder folienförmigen Darreichungsformen. Diese oral zu verabreichenden Medikamente können auf einem wasserlöslichen Polymer basieren, so daß der Arzneistoff bei Kontakt der Darreichungsform mit Speichel schnell freigesetzt wird. Wasserunlösliche oder schlecht wasserlösliche Polymere, vorzugsweise mit mukoadhäsiven Eigenschaften, werden für film- oder folienförmige Darreichungsformen mit einer retardierten Freisetzung und/oder zur transmukosalen Verabreichung des Arzneistoffs im Mundraum verwendet.

Arzneistoffhaltige Polymerfilme für orale oder transdermale therapeutische Systeme werden im Allgemeinen hergestellt, indem eine Beschichtungsmasse, umfassend das matrixbildende Polymer oder Polymergemisch und den Arzneistoff, in einer definierten Dicke auf einer Unterlage ausgestrichen und anschließend getrocknet wird. Die Beschichtung der Unterlage und die Trocknung der Beschichtung erfolgt üblicherweise in einem kontinuierlichen Prozeß. Unter Trocknung wird in diesem Zusammenhang generell das Entfernen der Lösemittel verstanden.

Bei der Beschichtungsmasse handelt es sich um eine Lösung oder Suspension, die das matrixbildende Polymer oder das matrixbildende Polymergemisch, mindestens einen Arzneistoff und gegebenenfalls weitere Hilfsstoffe, beispielsweise Permeationsenhancer, Weichmacher, Geschmacksstoffe, Farbstoffe, Konservierungsmittel, Antioxidantien oder dergleichen in einem Lösungsmittel enthalten. Vorzugsweise handelt es sich bei dem Lösungsmittel um ein organisches Lösungsmittel oder um ein Gemisch organischer Lösungsmittel.

Der Arzneistoff selbst kann in dem Lösungsmittel vollständig gelöst sein, muß es aber nicht. In den Fällen, in denen der Arzneistoff vollständig in dem Lösungsmittel gelöst sein soll, besteht schon bei geringem Überschreiten der Sättigungslöslichkeit für den Arzneistoff in der Beschichtungsmasse die Gefahr, daß sich während des Beschichtens der Unterlage Kristallisationskeime in der Beschichtungsmasse bilden. Solche Kristallisationskeime können sich im Beschichtungswerk durch eine lokal stärkere Verdunstung des Lösungsmittels oder einer Lösungsmittelkomponente des Lösungsmittelgemisches und einer dadurch verursachten lokalen Kristallisation des Arzneistoffs bilden.

Die lokale Kristallisation des Arzneistoffs in der Beschichtungsmasse ist kein Problem, wenn der getrocknete, lösungsmittelfreie Polymerfilm an Arzneistoff untersättigt ist, da sich unter diesen Bedingungen die Kristallisationskeime innerhalb kurzer Zeit auflösen. Die lokale Kristallisation des Arzneistoffs in der Beschichtung stellt jedoch ein Problem dar, wenn auch der getrocknete Polymerfilm an Arzneistoff übersättigt ist oder Arzneistoff in einer amorphen Modifikation enthält. Mit Arzneistoff übersättigte Matrixsysteme, und dazu gehören auch Systeme, die den Arzneistoff in amorpher Form enthalten, haben den Vorteil einer besonders hohen thermodynamischen Aktivität und Bioverfügbarkeit des Arzneistoffs. Diesem Vorteil steht jedoch der Nachteil entgegen, daß die übersättigten Matrixsysteme metastabil sind und die Bioverfügbarkeit des Arzneistoffs durch seine Kristallisation stark beeinträchtigt ist.

Um die Bildung von kristallinen Hydraten von bei Raumtemperatur flüssigen Wirkstoffen in einer polymeren Matrixschicht zu verhindern, wurde in der US 4,832,953 vorgeschlagen, die arzneistoffhaltige Polymermatrix zu erwärmen. Speziell wird die Bildung von Scopolamin-Hydrat-Kristallen in nicht-wäßrigen, polymeren Matrices und eine damit verbundene signifikant nachteilige Wirkung auf die Freisetzungsgeschwindigkeit von Scopolamin aus den Verabreichungseinheiten beschrieben, bei denen flüssige Scopolamin-Base in einer Polyisobutylen-Mineralöl-Matrix dispergiert vorliegt. Zur Lösung dieses Problems wird vorgeschlagen, die fertig verpackten Verabreichungseinheiten für eine Dauer von 24 Stunden auf 60 °C zu erwärmen. Diese Erwärmung der Verabreichungseinheiten war ausreichend, um in der Matrix vorhandene Kristalle des Hydrats der Scopolamin-Base mit einem Schmelzpunkt von 59 °C zu schmelzen und eine Bildung von Kristallen nach dem Abkühlen der Verabreichungseinheiten zu verhindern.

Allerdings konnte bei derart behandelten Verabreichungseinheiten das Auftreten zusätzlicher Kristalle beobachtet werden, die eine höhere Schmelztemperatur von 67-70 °C aufwiesen. Diese zusätzlichen Kristalle konnten durch die Erwärmung der Verabreichungseinheiten auf 60 °C für 24 Stunden nicht beseitigt werden. Auch durch eine Erhöhung der Temperatur, auf die die verpackten Verabreichungseinheiten erwärmt wurden, konnten diese zusätzlichen Kristalle nicht beseitigt werden. Zur Lösung dieses Problems wird mit der US 5,662,928 vorgeschlagen, die Scopolamin enthaltende Matrixschicht - zusätzlich zu der Wärmebehandlung der fertiggestellten und bereits verpackten Verabreichungseinheiten - unmittelbar vor dem Zusammenlaminieren mit der die Freisetzung kontrollierenden Membran für die Dauer von 5 bis 15 Minuten auf eine Temperatur von zwischen 67 °C und 90 °C zu erwärmen. "Unmittelbar" bedeutet gemäß US 5,662,928, daß das Zusammenlaminieren innerhalb von 24 Stunden, besser noch innerhalb von 18 Stunden, nach Aufbringen der Scopolaminbeschichtung erfolgen muß.

Dieses bekannte Verfahren zur Vermeidung einer Kristallisation des Arzneistoffs in der Polymermatrix weist den Nachteil auf, daß die Matrixschicht nach ihrer Beschichtung innerhalb kurzer Zeit (24 Stunden) weiterverarbeitet werden muß und nicht länger zwischengelagert werden kann. Diese weitere Wärmebehandlung ist ein zusätzlicher Herstellschritt und macht das Herstellungsverfahren zeitintensiv und somit auch kostenintensiv.

Die Aufgabe, die der vorliegenden Erfindung zugrunde lag, bestand darin, einen einfachen und kostengünstigen Weg zu finden, die Kristallisation von Rotigotin in einer mit diesem Arzneistoff übersättigten Polymermatrix zu verhindern.

Die Aufgabe wird in überraschend einfacher Weise dadurch gelöst, daß die für die Herstellung des Polymerfilms ausgestrichene lösungsmittelhaltige Beschichtungsmasse, umfassend ein matrixbildendes Polymer oder Polymergemisch und Rotigotin als Arzneistoff, der in Form einer festen Lösung in dem matrixbildenden Polymer dispergiert ist, bei Temperaturen getrocknet wird, die nicht länger als 15 Minuten bei 10 bis 25 °C über der Schmelztemperatur des in der Beschichtungsmasse enthaltenen Arzneistoffs liegen.

Bei der vorliegenden Erfindung handelt es sich somit um ein Verfahren zur Verhinderung der Kristallisation des Arzneistoffs Rotigotin in einem Rotigotin-haltigen Polymerfilm, der für die Herstellung transdermaler therapeutischer Systeme oder oral zu verabreichender Medikamente geeignet ist. Das Verfahren zeichnet sich dadurch aus, daß eine Unterlage mit einer Beschichtungsmasse, umfassend ein Lösungsmittel oder Lösungsmittelgemisch, ein matrixbildendes Polymer oder Polymergemisch und Rotigotin, beschichtet und das Lösungsmittel oder Lösungsmittelgemisch unter Anwendung von Wärme aus der Beschichtung entfernt wird, wobei die Maximaltemperatur während des Entfernens des Lösungsmittels die Schmelztemperatur des Arzneistoffs zeitweise um 10 bis 25 °C übersteigt und damit höher als zur reinen Trocknung erforderlich ist.

Trocknungstemperaturen über 130 °C können jedoch problematisch sein, weil auch wärmeresistente Polyesterfilme, die häufig als Unterlage für die Beschichtung verwendet werden, bei diesen Temperaturen zu erweichen beginnen.

Die Trocknung der arzneistoffhaltigen Beschichtung erfolgt nicht länger als 15 Minuten bei 10 bis 25°C über der Schmelztemperatur des Arzneistoffs.

Die Rotigotin-haltigen Polymerfilme werden üblicherweise hergestellt, indem Rotigotin und gegebenenfalls weitere Hilfsstoffe einer Lösung oder Suspension des matrixbildenden Polymers oder Polymergemisches zugesetzt wird. Die so erhaltene Beschichtungsmasse wird auf einer folienförmigen Unterlage zu einer Beschichtung mit definierter Dicke ausgestrichen. Die beschichtete Unterlage wird anschließend durch einen Trockenkanal geführt, in dem das Lösungsmittel oder Lösungsmittelgemisch bei erhöhter Temperatur entfernt wird, so daß nur noch geringe Restemengen an Lösungsmittel, maximal 0,5 Gew.-%, in der Beschichtung zurückbleiben.

Der Arzneistoff Rotigotin ist in Form einer festen Lösung in dem matrixbildenden Polymer dispergiert. Unter "fester Lösung" wird eine molekulardisperse Verteilung des Arzneistoffs im Matrixpolymer verstanden.

Bei der Festlegung der Trocknungsbedingungen ist es ein Ziel, das/die Lösungsmittel unter möglichst schonenden Bedingungen zu entfernen. Das Lösungsmittel wird vom Fachmann in Abhängigkeit vom Matrixpolymer ausgewählt. Die gängigen Lösungsmittel sind Heptan, Hexan, Cyclohexan, Ethylacetat, Ethanol, Methanol, Isopropanol und Tetrahydrofuran.

Das matrixbildende Polymer selbst stellt keinen limitierenden Faktor für das erfindungsgemäße Verfahren dar. Als matrixbildende Polymere kommen beispielsweise Polysiloxane, Polyacrylate, Polyisobutylene, Blockcopolymere wie Styrol-Butadien-Styrol-Blockcopolymere und deren Mischungen in Betracht. Besonders bevorzugte matrixbildende Polymere sind aminresistente Polysiloxane.

Vorzugsweise handelt es sich bei den matrixbildenden Polymeren um haftklebende oder selbstklebende Polymere.

Als Arzneistoff wird Rotigotin eingesetzt.

### Beispiel 1 (Vergleichsbeispiel)

Es wurde ein zweiphasiges System mit einer äußeren Phase aus einem selbstklebenden Polysiloxanpolymer und einer inneren Phase aus einem Polyvinylpyrrolidon/Arzneistoffkomplex hergestellt. Die Beschichtungsmasse bestand aus einer Dispersion, bei der der Polysiloxankleber der äußeren Phase in n-Heptan und der Arzneistoff Rotigotin sowie das Polyvinylpyrrolidon der inneren Phase in Ethanol gelöst vorlagen. Der Arzneistoff Rotigotin hat eine Schmelztemperatur von 97 - 99 °C und die Sättigungslöslichkeit in der Beschichtungsmasse ist bei Raumtemperatur überschritten.

Die Trocknung der Beschichtung erfolgte in einem Trockenkanal mit dem in Tabelle 1 angegebenen Temperaturprofil.

**Tabelle 1: Temperaturprofil für die Trocknung eines Rotigotin enthaltenden Polymerfilms.**

| | | | | | | |
|---|---|---|---|---|---|---|
| Temperatur [°C] | 40 | 50 | 55 | 60 | 70 | 80 |
| Zeit [s] | 50 | 50 | 50 | 50 | 70 | 70 |

Infolge der Beschichtung entstanden zahlreiche Kristallisationskeime in dem Polymerfilm, die unmittelbar nach der Beschichtung nicht sichtbar waren. Bereits 24 Stunden nach seiner Trocknung zeigte der Polymerfilm eine mikroskopisch sichtbare Kristallisation des Arzneistoffs innerhalb der Matrix. Nach 2 Tagen war die Kristallisation des Arzneistoffs im gesamten Laminat auch mit bloßem Auge zu erkennen.

### Beispiel 2

Es wurde eine rotigotinhaltige Beschichtungsmasse wie in Beispiel 1 beschrieben hergestellt und in gleicher Weise auf eine Unterlage ausgestrichen. Abweichend von Beispiel 1 wurde diese Beschichtung mit dem in Tabelle 2 angegebenen Temperaturprofil getrocknet.

**Tabelle 2: Temperaturprofil für die Trocknung eines Rotigotin enthaltenden Polymerfilms gemäß dem erfindungsgemäßen Verfahren.**

| | | | | | | | | | |
|---|---|---|---|---|---|---|---|---|---|
| Temperatur [°C] | 40 | 50 | 60 | 70 | 80 | 90 | 100 | 105 | 115 |
| Zeit [s] | 50 | 25 | 25 | 25 | 25 | 25 | 25 | 50 | 100 |

Bei diesem Trocknungsverfahren wurde die Beschichtung am Ende der Trocknung für die Dauer von knapp 3 Minuten einer Temperatur ausgesetzt, die über der Schmelztemperatur des Arzneistoffs von 97-99 °C lag.

Bei dem arzneistoffhaltigen Polymerfilm, der gemäß dem in Tabelle 2 angegebenen Temperaturprofil getrocknet wurde, war auch 2 Jahre nach der Beschichtung keine Kristallisation des Arzneistoffs in der Polymermatrix festzustellen.

Die höhere Trocknungstemperatur bei dem Verfahren nach Beispiel 2 hat die Kristallisation von Rotigotin in der Polymermatrix als Folge der Bildung von Saatkristallen während der Beschichtung vollständig unterbunden.

## Patentansprüche

1. Verfahren zur Verhinderung der Kristallisation des Arzneistoffs Rotigotin in einem Polymerfilm, **dadurch gekennzeichnet, dass** die für die Herstellung des Polymerfilms ausgestrichene lösungsmittelhaltige Beschichtungsmasse, umfassend ein matrixbildendes Polymer oder Polymergemisch und Rotigotin als Arzneistoff, der in Form einer festen Lösung in dem matrixbildenden Polymer dispergiert ist, bei Temperaturen getrocknet wird, die nicht länger als 15 Minuten bei 10 bis 25 °C über der Schmelztemperatur des in der Beschichtungsmasse enthaltenen Arzneistoffs liegen.

2. Verfahren nach Anspruch 1, **dadurch gekennzeichnet, dass** die Temperaturen für mindestens 1 Minute 15 bis 25 °C über der Schmelztemperatur des Arzneistoffs liegen.

3. Verfahren nach Anspruch 1 oder 2, **dadurch gekennzeichnet, dass** die Trocknungstemperatur 130 °C nicht übersteigt.

4. Verfahren nach einem der vorangehenden Ansprüche, **dadurch gekennzeichnet, dass** die beschichtete Masse für mindestens 100 Sekunden und bevorzugt für mindestens 3 Minuten, bei der 10 bis 25 °C über dem Schmelzpunkt des Arzneistoffs liegenden Temperatur getrocknet wird.

5. Verfahren nach einem der vorangehenden Ansprüche, **dadurch gekennzeichnet, dass** die beschichtete Masse nicht länger als 10 Minuten und bevorzugt nicht länger als 5 Minuten, bei der 10 bis 25 °C über dem Schmelzpunkt des Arzneistoffs liegenden Temperatur getrocknet wird.

6. Verfahren nach einem der vorangehenden Ansprüche, **dadurch gekennzeichnet, dass** das matrixbildende Polymer oder mindestens eines der Polymere des matrixbildenden Polymergemisches aus der Gruppe ausgewählt ist, die aus Polysiloxanen, Polyacrylaten, Polyisobutylenen und BlockCopolymeren besteht.

7. Verfahren nach einem der vorhergehenden Ansprüche, **dadurch gekennzeichnet, dass** das matrixbildende Polymer ein aminresistentes Polysiloxan ist.

8. Verfahren nach einem der vorhergehenden Ansprüche, **dadurch gekennzeichnet, dass** das Lösungsmittel aus der Gruppe von organischen Lösungsmitteln ausgewählt ist, die aus Heptan, Hexan, Cyclohexan, Ethylacetat, Ethanol, Methanol, Isopropanol und Tetrahydrofuran besteht.

9. Verfahren nach einem der vorhergehenden Ansprüche, **dadurch gekennzeichnet, dass** der Arzneistoff eine Schmelztemperatur von unter 105 °C.

## Claims

1. Method for preventing the crystallization of the pharmaceutical rotigotine in a polymer film, **characterized in that** the solvent-containing coating material applied for producing the polymer film, comprising a matrix-forming polymer or polymer mixture and rotigotine as pharmaceutical, which is dispersed in the form of a solid solution in the matrix-forming polymer, is dried at temperatures which are 10 to 25 °C above the melting temperature of the pharmaceutical present in the coating material for not longer than 15 minutes.

2. Method according to Claim 1, **characterized in that** the temperatures are 15 to 25 °C above the melting temperature of the pharmaceutical for at least 1 minute.

3. Method according to Claim 1 or 2, **characterized in that** the drying temperature does not exceed 130 °C.

4. Method according to any of the preceding claims, **characterized in that** the coated material is dried for at least 100 seconds, and preferably for at least 3 minutes, at the temperature which is 10 to 25 °C above the melting point of the pharmaceutical.

5. Method according to any of the preceding claims, **characterized in that** the coated material is dried for not longer than 10 minutes, and preferably not longer than 5 minutes, at the temperature which is 10 to 25 °C above the melting point of the pharmaceutical.

6. Method according to any of the preceding claims, **characterized in that** the matrix-forming polymer or at least one of the polymers of the matrix-forming polymer mixture is selected from the group consisting of polysiloxanes, polyacrylates, polyisobutylenes, and block copolymers.

7. Method according to any of the preceding claims, **characterized in that** the matrix-forming polymer is an amine-resistant polysiloxane.

8. Method according to any of the preceding claims, **characterized in that** the solvent is selected from the group of organic solvents consisting of heptane, hexane, cyclohexane, ethyl acetate, ethanol, methanol, isopropanol, and tetrahydrofuran.

9. Method according to any of the preceding claims, **characterized in that** the pharmaceutical a melting temperature of below 105 °C.

## Revendications

1. Procédé pour empêcher la cristallisation de la substance pharmaceutique rotigotine dans un film polymère, **caractérisé en ce que** la masse de revêtement contenant un solvant, étalée pour la production du film polymère, comprenant un polymère ou un mélange de polymères formant une matrice et de la rotigotine en tant que substance pharmaceutique, qui est dispersée sous la forme d'une solution solide dans le polymère formant une matrice, est séchée à des températures qui ne se situent pas pendant plus de 15 minutes de 10 à 25°C au-dessus de la température de fusion de la substance pharmaceutique contenue dans la masse de revêtement.

2. Procédé selon la revendication 1, **caractérisé en ce que** les températures se situent pendant au moins 1 minute de 15 à 25°C au-dessus de la température de fusion de la substance pharmaceutique.

3. Procédé selon la revendication 1 ou 2, **caractérisé en ce que** la température de séchage ne dépasse pas 130°C.

4. Procédé selon l'une des revendications précédentes, **caractérisé en ce que** la masse revêtue est séchée pendant au moins 100 secondes et de préférence pendant au moins 3 minutes, à la température se situant de 10 à 25°C au-dessus du point de fusion de la substance pharmaceutique.

5. Procédé selon l'une des revendications précédentes, **caractérisé en ce que** la masse revêtue est séchée pendant une durée ne dépassant pas 10 minutes et de préférence, ne dépassant pas 5 minutes, à la température se situant de 10 à 25°C au-dessus du point de fusion de la substance pharmaceutique.

6. Procédé selon l'une des revendications précédentes, **caractérisé en ce que** le polymère formant une matrice ou au moins l'un des polymères du mélange de polymères formant une matrice est choisi dans le groupe qui est constitué de polysiloxanes, de polyacrylates, de polyisobutylènes et de copolymères en bloc.

7. Procédé selon l'une des revendications précédentes, **caractérisé en ce que** le polymère formant une matrice est un polysiloxane résistant à une amine.

8. Procédé selon l'une des revendications précédentes, **caractérisé en ce que** le solvant est choisi dans le groupe des solvants organiques qui est constitué de l'heptane, l'hexane, le cyclohexane, l'acétate d'éthyle, l'éthanol, le méthanol, l'isopropanol et le tétrahydrofurane.

9. Procédé selon l'une des revendications précédentes, **caractérisé en ce que** la substance pharmaceutique a une température de fusion, inférieure à 105°C.
